# EUROPEAN PATENT APPLICATION

(11) **EP 4 628 333 A1**
(43) Date of publication of application: **08.10.2025**
(21) Application number: 23897346.5
(22) Date of filing: 30.10.2023
(51) Int. Cl.: B60H 3/06, B60H 1/00, F24F 8/26

(54) **AIR CONDITIONING SYSTEM**

(30) Priority: 30.11.2022 JP 2022192467
(71) Applicant: Panasonic Intellectual Property Management Co., Ltd., Kadoma-shi, Osaka 571-0057 (JP)
(72) Inventor: MATSUURA Haruto, Kadoma-shi, Osaka 571-0057 (JP)
(74) Representative: Grünecker Patent- und Rechtsanwälte PartG mbB
(86) International application number: PCT/JP2023/039027
(87) International publication number: WO 2024/116690

(57) **Abstract**

The present disclosure provides an air conditioning system that performs bacteria inactivation on an evaporator more satisfactorily. The air conditioning system (2) according to the present device includes a blower (4), an evaporator (5), a case (20), and a functional substance generator (3). The blower (4) generates a flow of air toward a vehicle interior. The evaporator (5) is disposed in the middle of a flow path of air and exchanges heat of air. The case (20) accommodates the blower (4) and the evaporator (5). The functional substance generator (3) generates a functional substance. The functional substance generator (3) is disposed upstream of evaporator (5) in a first direction which is a flow direction of the air toward the vehicle interior.

## Description

### TECHNICAL FIELD

The present disclosure generally relates to air conditioning systems, and more particularly relates to an air conditioning system including an evaporator.

### BACKGROUND ART

PTL 1 discloses a vehicle air conditioner including an HVAC unit. HVAC is an abbreviation of Heating Ventilating Air-Conditioning. The HVAC unit of PTL 1 includes a housing, a blower, an evaporator, and an ion generator. The blower, the evaporator, and the ion generator are disposed in the housing. The ion generator is disposed downstream of the evaporator and generates negative ions and ozone.

The vehicle air conditioner of PTL 1 performs an ion dust removal mode and an ozone bacteria inactivation mode. In the ion dust removal mode, the blower operates, and negative ions are supplied into the vehicle interior by the operation of the blower to remove floating fine particles in the vehicle interior. In the ozone bacteria inactivation mode, the blower stops, and ozone removes bacilli and fungi (that is, performs bacteria inactivation) attached to the outer surface of the evaporator or the inner surface of the housing.

### Citation List

### Patent Literature

PTL 1: Unexamined Japanese Patent Publication No. 2010-042750

### SUMMARY OF THE INVENTION

### Technical problem

In the ozone bacteria inactivation mode of the vehicle air conditioner of PTL 1, since only ozone is accumulated downstream of the evaporator, bacteria inactivation of the evaporator may be insufficient.

The present disclosure has been made in view of the above circumstances, and an object of the present disclosure is to provide an air conditioning system in which bacteria inactivation on an evaporator is performed more satisfactorily.

### Solution to problem

An air conditioning system according to an aspect of the present disclosure includes a blower, an evaporator, a case, and a functional substance generator. The blower generates a flow of air toward the vehicle interior. The evaporator is disposed in the middle of a flow path of the air and exchanges heat of the air. The case accommodates the blower and the evaporator. The functional substance generator generates a functional substance. The functional substance generator is disposed upstream of the evaporator in a first direction which is a flow direction of the air toward the vehicle interior.

### Advantageous effect of invention

In the air conditioning system of the above aspect of the present disclosure, bacteria inactivation of the evaporator can be performed more satisfactorily.

### BRIEF DESCRIPTION OF THE DRAWINGS

Fig. 1 is a schematic view of a vehicle including an air conditioning system according to a first exemplary embodiment.
Fig. 2 is a schematic view illustrating an instrument panel of the vehicle according to the first exemplary embodiment.
Fig. 3 is a schematic view illustrating a configuration of the air conditioning system according to the first exemplary embodiment.
Fig. 4 is a flowchart illustrating an operation of the air conditioning system according to the first exemplary embodiment.
Fig. 5 is a flowchart illustrating an operation in an exposure mode of the air conditioning system according to the first exemplary embodiment.
Fig. 6 is a flowchart illustrating an operation in a drying mode of the air conditioning system according to the first exemplary embodiment.
Fig. 7 is a flowchart illustrating an operation in a filling mode of the air conditioning system according to the first exemplary embodiment.
Fig. 8 is a schematic view illustrating a configuration of an air conditioning system according to a first modification of the first exemplary embodiment.
Fig. 9 is a schematic view illustrating a configuration of an air conditioning system according to a second modification of the first exemplary embodiment.
Fig. 10 is a schematic view illustrating a configuration of an air conditioning system according to a third modification of the first exemplary embodiment.
Fig. 11 is a schematic view illustrating a configuration of an air conditioning system according to a fourth modification of the first exemplary embodiment.
Fig. 12 is a schematic view illustrating a configuration of an air conditioning system according to a fifth modification of the first exemplary embodiment.
Fig. 13 is a schematic view illustrating a configuration of an air conditioning system according to a second exemplary embodiment.
Fig. 14 is a flowchart illustrating an operation in an exposure mode of the air conditioning system according to the second exemplary embodiment.
Fig. 15 is a flowchart illustrating an operation in a drying mode of the air conditioning system according to the second exemplary embodiment.
Fig. 16 is a flowchart illustrating an operation in a filling mode of the air conditioning system according to the second exemplary embodiment.
Fig. 17 is a schematic view illustrating a configuration of an air conditioning system according to a first modification of the second exemplary embodiment.
Fig. 18 is a schematic view illustrating a configuration of an air conditioning system according to a second modification of the second exemplary embodiment.
Fig. 19 is a schematic view illustrating a configuration of an air conditioning system according to a third modification of the second exemplary embodiment.

### DESCRIPTION OF EMBODIMENT

Hereinafter, preferred exemplary embodiments of the present disclosure will be described in detail with reference to the drawings. In the exemplary embodiments described below, elements common to each other are denoted by the same reference numerals, and redundant description of the common elements may be omitted.

The following exemplary embodiments are merely ones of various exemplary embodiments of the present disclosure. The exemplary embodiments can be variously changed according to the design, as long as the object of the present disclosure can be achieved. A plurality of exemplary embodiments (including modifications) may be implemented by being appropriately combined.

The drawings described in the present disclosure are schematic views, and ratios of sizes and thicknesses of the elements in the drawings do not necessarily reflect actual dimensional ratios.

### (First exemplary embodiment)

### (1) Overview

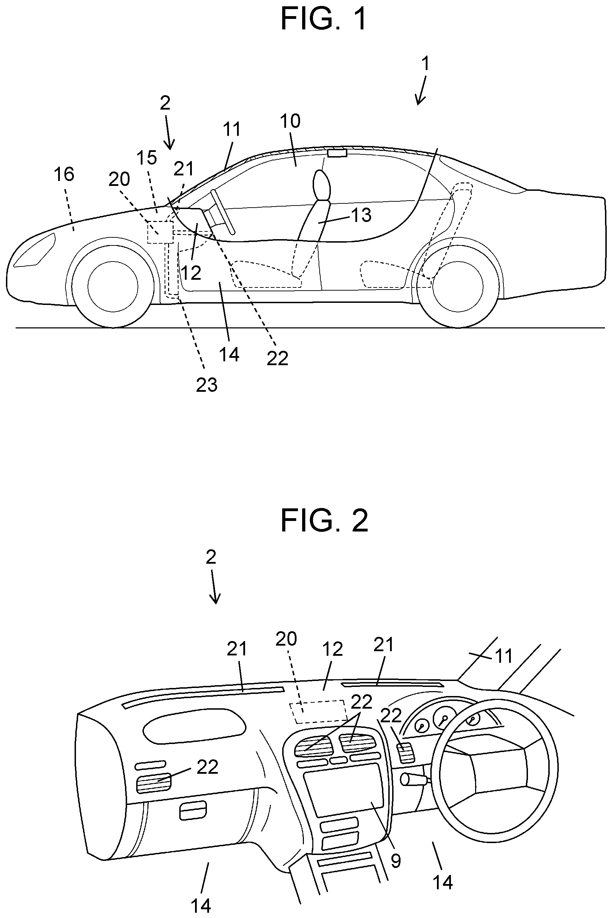

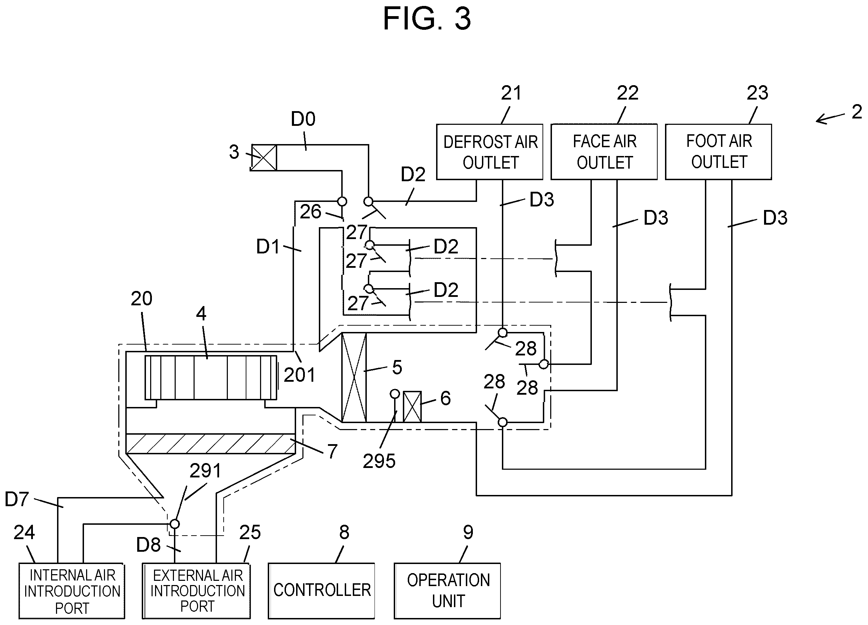

First, an overview of air conditioning system 2 according to the first exemplary embodiment will be described with reference to Figs. 1 to 3. Fig. 1 is a schematic view of vehicle 1 including air conditioning system 2 according to the first exemplary embodiment. Fig. 2 is a schematic view illustrating instrument panel 12 of vehicle 1 according to the first exemplary embodiment. Fig. 3 is a schematic view illustrating a configuration of air conditioning system 2 according to the first exemplary embodiment.

As illustrated in Figs. 1 and 2, air conditioning system 2 is used in vehicle 1 such as an automobile driven by a person. Air conditioning system 2 is provided in device room 15 of vehicle 1 and performs a cooling operation for sending cool air into vehicle interior 10 of vehicle 1, a heating operation for sending warm air into vehicle interior 10. The "vehicle" in the present disclosure includes a passenger car, a large vehicle such as a truck or a bus, a train, an electric cart, and a construction machine.

As illustrated in Fig. 3, air conditioning system 2 includes blower 4, evaporator 5, case 20, and functional substance generator 3.

Blower 4 generates a flow of air toward vehicle interior 10 (see Fig. 1).

Evaporator 5 is disposed in the middle of the air flow path and exchanges heat with air passing through evaporator 5. More specifically, heat exchange is performed between the air drawn into air conditioning system 2 by blower 4 and the evaporator. For example, in the case of cooling, the air drawn into air conditioning system 2 is cooled through heat exchange when passing through the evaporator, becomes cool air, and is discharged to the outside of the air conditioning system.

Case 20 accommodates blower 4 and evaporator 5.

Functional substance generator 3 generates a functional substance. The "functional substance" in the present disclosure includes charged fine water particles, radicals, and air ions. The functional substance has a useful effect in various scenes in addition to effects such as bacteria inactivation, odor removal, moisture keeping, freshness keeping, and virus inactivation. In the following description, "bacteria inactivation" is a representative effect of the functional substance. Functional substance generator 3 in the first exemplary embodiment generates charged fine water particles having a nanometer size and containing radicals. However, functional substance generator 3 may be an ion generator or an ozone generator. Functional substance generator 3 may be a device capable of generating a plurality of functional substances among charged fine water particles, ions (for example, negative ions), and ozone. The charged fine water particles may be a liquid other than water.

Functional substance generator 3 is disposed upstream of evaporator 5 in a first direction which is a flow direction of air toward vehicle interior 10.

The "first direction" in the present disclosure is a flow direction of air from internal air introduction port 24 or external air introduction port 25, which will be described later, toward blower 4 and from blower 4 toward vehicle interior 10. In the first direction, blower 4 is located upstream of evaporator 5.

The meaning that "functional substance generator 3 is disposed upstream of evaporator 5" includes the case where functional substance generator 3 itself is disposed upstream of evaporator 5 in the air flow direction and the case where the functional substances generated by functional substance generator 3 join upstream of evaporator 5 in the air flow direction. In the example of Fig. 3, functional substance generator 3 is disposed downstream of blower 4 and upstream of evaporator 5 in the first direction.

The functional substance generated by functional substance generator 3 travels toward evaporator 5 with the flow of air (that is, together with the air flow,) at the time of the operation of blower 4, and then passes through evaporator 5.

In air conditioning system 2 according to the first exemplary embodiment, at the time of the operation of blower 4, the functional substance flows toward evaporator 5, and then passes through evaporator 5. Thus, evaporator 5 is easily exposed to the functional substance. For example, even when evaporator 5 is a large evaporator, the functional substance passes through evaporator 5, and thus the surface of evaporator 5 can be entirely exposed to the functional substance. In this manner, in air conditioning system 2 according to the first exemplary embodiment, bacteria inactivation on evaporator 5 can be performed more satisfactorily.

In air conditioning system 2 according to the first exemplary embodiment, the bacteria inactivation on evaporator 5 can be performed at the time of the operation of blower 4, that is, during the cooling operation or the heating operation of air conditioning system 2.

When the functional substance generated by functional substance generator 3 is charged fine water particles having a nanometer size, the functional substance easily passes through evaporator 5 as compared with the case where the functional substance is an ion. That is, when the functional substance is charged fine water particles having a nanometer size, there is an advantage that bacteria inactivation on evaporator 5 can be performed more satisfactorily and the functional substance easily reaches the inside of vehicle interior 10.

### (2) Details

Hereinafter, a detailed configuration of vehicle 1 including air conditioning system 2 according to the first exemplary embodiment will be described with reference to Figs. 1 to 3.

### (2.1) Configuration of vehicle

As illustrated in Fig. 1, vehicle 1 includes air conditioning system 2, vehicle interior 10, windshield 11, instrument panel 12, a plurality of seats 13 including a driver seat and a passenger seat, foot space 14, device room 15, and engine room 16.

Foot space 14 is a space in front of driver seat and the passenger seat and below instrument panel 12. Foot space 14 is a space where the feet of the user sitting on the driver seat or the passenger seat is located.

Device room 15 is partitioned from vehicle interior 10 by instrument panel 12. Case 20 of air conditioning system 2 is disposed in device room 15. Engine room 16 is partitioned from device room 15 by a wall.

As illustrated in Fig. 2, instrument panel 12 is provided with a plurality of defrost air outlets 21, a plurality of face air outlets 22, and an operation unit 9. In the drawings other than Fig. 2, only one of the plurality of defrost air outlets 21 is illustrated, and only one of the plurality of face air outlets 22 is illustrated. In the first exemplary embodiment, the plurality of defrost air outlets 21, the plurality of face air outlets 22, and the operation unit 9 are components of air conditioning system 2.

### (2.2) Configuration of air conditioning system

As illustrated in Fig. 3, air conditioning system 2 includes case 20, a plurality of defrost air outlets 21, a plurality of face air outlets 22, a plurality of foot air outlets 23, internal air introduction port 24, external air introduction port 25, functional substance generator 3, blower 4, evaporator 5, heater 6, filter 7, controller 8, and operation unit 9. Fig. 3 illustrates one defrost air outlet 21 among the plurality of defrost air outlets 21. Similarly, in Fig. 3, one face air outlet among the plurality of face air outlets 22 is illustrated. Similarly, in Fig. 3, one foot air outlet among the plurality of foot air outlets 23 is illustrated.

Air conditioning system 2 also includes functional substance duct D0, case duct D1, a plurality of vehicle-interior ducts D2, a plurality of air ducts D3, internal-air duct D7, and external-air duct D8. In the first exemplary embodiment, three vehicle-interior ducts D2 are provided. In the first exemplary embodiment, three air ducts D3 are provided.

Air conditioning system 2 also includes case valve 26, a plurality of vehicle-interior valves 27, a plurality of air duct valves 28, an introduction port valve 291, and a heater valve 295. In the first exemplary embodiment, three vehicle-interior valves 27 are provided. In the first exemplary embodiment, three air duct valves 28 are provided. In the following description, each of the plurality of vehicle-interior valves 27 may be simply referred to as "vehicle-interior valve 27", and each of the plurality of air duct valves 28 may be simply referred to as "air duct valve 28". Case valve 26, vehicle-interior valve 27, air duct valve 28, introduction port valve 291, and heater valve 295 are controlled by controller 8.

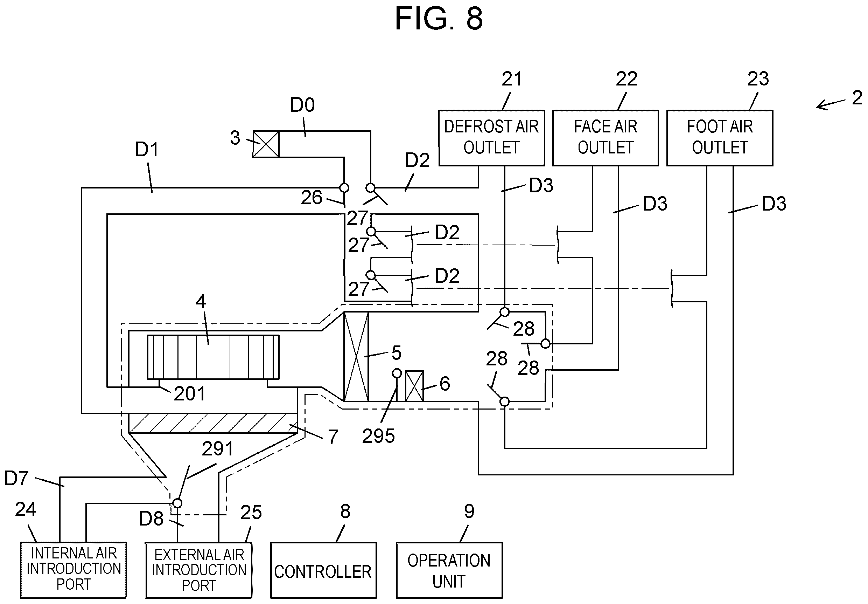

Case 20 of the present disclosure is a portion surrounded by a two-dot chain line in Figs. 3, 8 to 13, and 17 to 19. Figs. 8 to 13 and Figs. 17 to 19 will be described in detail later. Case 20 accommodates blower 4, evaporator 5, heater 6, and filter 7. Case 20 is provided with a plurality of air duct valves 28, introduction port valve 291, and heater valve 295.

Case 20 has junction port 201. Junction port 201 is formed upstream of evaporator 5 and downstream of blower 4 in the first direction. Junction port 201 is connected with case duct D1. That is, case 20 is connected to functional substance generator 3 via case duct D1 and functional substance duct D0. When functional substance generator 3 is operated and case valve 26 is open, the functional substances generated by functional substance generator 3 join between blower 4 and evaporator 5 in case 20. That is, the functional substance generated by functional substance generator 3 joins at a position downstream of blower 4 and upstream of evaporator 5 in the first direction.

Case 20 is connected to defrost air outlet 21, face air outlet 22, and foot air outlet 23 via the plurality of air ducts D3. Case 20 is connected to internal air introduction port 24 via internal-air duct D7, and is connected to external air introduction port 25 via external-air duct D8.

Introduction port valve 291 is a valve that can plug (that is, close) one of internal-air duct D7 connected to internal air introduction port 24 and external-air duct D8 connected to external air introduction port 25.

The plurality of air duct valves 28 can independently plug air duct D3 connected to defrost air outlet 21, air duct D3 connected to face air outlet 22, and air duct D3 connected to foot air outlet 23. That is, air duct valve 28 is a valve that opens and closes the flow path of air duct D3.

As illustrated in Figs. 1 and 2, defrost air outlet 21 is open such that air or the functional substance flowing out into vehicle interior 10 is directed toward windshield 11 or the periphery of windshield 11.

As illustrated in Figs. 1 and 2, face air outlet 22 is open such that air or the functional substance flowing out into vehicle interior 10 is directed toward an upper body of the user sitting on the driver seat or the passenger seat.

As illustrated in Fig. 1, foot air outlet 23 is open such that air or the functional substance flowing out into vehicle interior 10 is directed toward the feet of the user sitting on the driver seat or the user sitting on the passenger seat.

Internal air introduction port 24 and external air introduction port 25 illustrated in Fig. 3 are introduction ports for introducing air into case 20. Internal air introduction port 24 is open toward vehicle interior 10, for example. That is, internal air introduction port 24 is an introduction port for introducing air from vehicle interior 10 into case 20. External air introduction port 25 is open toward the outside of the vehicle, for example. That is, external air introduction port 25 is an introduction port for introducing air from the outside of the vehicle into case 20.

Functional substance duct D0 is a duct through which the functional substance generated by functional substance generator 3 passes. Functional substance generator 3 is disposed at one end of functional substance duct D0. The other end of functional substance duct D0 is connected to case duct D1 and vehicle-interior duct D2.

Case duct D1 is a duct through which the functional substance generated by functional substance generator 3 passes. One end of case duct D1 is connected to functional substance duct D0. The other end of case duct D1 is connected to junction port 201 of case 20. That is, case duct D1 is a duct connecting functional substance generator 3 and case 20.

Vehicle-interior duct D2 is a duct through which the functional substance generated by functional substance generator 3 passes. One end of vehicle-interior duct D2 is connected to functional substance duct D0. The other end of vehicle-interior duct D2 is connected to air duct D3. That is, vehicle-interior duct D2 is a duct connecting functional substance generator 3 and air duct D3.

Air duct D3 is disposed downstream of evaporator 5 in the first direction. Air duct D3 is a duct through which an air flow generated by blower 4 or the functional substance generated by functional substance generator 3 passes. One end of air duct D3 is connected to case 20. The other end of air duct D3 is connected to any one of defrost air outlet 21, face air outlet 22, and foot air outlet 23. That is, air duct D3 is a duct connecting vehicle interior 10 and case 20. Air duct D3 is connected to the other end of vehicle-interior duct D2 between one end and the other end of air duct D3.

One end of internal-air duct D7 is connected to case 20. The other end of internal-air duct D7 is connected to internal air introduction port 24. That is, internal-air duct D7 is a duct connecting case 20 and internal air introduction port 24.

One end of external-air duct D8 is connected to case 20. The other end of external-air duct D8 is connected to external air introduction port 25. That is, external-air duct D8 is a duct connecting case 20 and external air introduction port 25.

Case valve 26 is provided at one end of case duct D1. Case valve 26 is a valve that can plug (that is, close) case duct D1. That is, case valve 26 is a valve that opens and closes the flow path of case duct D1.

Vehicle-interior valve 27 is provided at one end of vehicle-interior duct D2. Vehicle-interior valve 27 is a valve that can plug (that is, close) vehicle-interior duct D2. That is, vehicle-interior valve 27 is a valve that opens and closes the flow path of vehicle-interior duct D2.

Blower 4 generates a flow of air. Blower 4 includes a motor and operates when a shaft of the motor rotates. Blower 4 operates under the control of controller 8.

Blower 4 in the first exemplary embodiment is configured to generate a flow of air in the first direction and a flow of air in a second direction opposite to the first direction. The "second direction" in the present disclosure is a flow direction of air from vehicle interior 10 toward blower 4 and from blower 4 toward internal air introduction port 24 or external air introduction port 25. In the first direction, blower 4 is located upstream of evaporator 5. In the second direction, blower 4 is located upstream of filter 7, and heater 6 is located upstream of evaporator 5.

Blower 4 in the first exemplary embodiment can change the air pressure and the air volume of the air output from blower 4. For example, blower 4 is configured to output air at a first air pressure or a second air pressure. The second air pressure is higher than the first air pressure. The first air pressure and the second air pressure do not need to be constant air pressures as long as they are air pressures within predetermined ranges. For example, blower 4 is configured to output air with a first air volume or a second air volume. The second air volume is larger than the first air volume. The first air volume and the second air volume do not need to be constant as long as they are air volumes within predetermined ranges. Although only the case where blower 4 changes the air pressure is illustrated in the following description, blower 4 may change the air volume instead of the air pressure or in addition to the air pressure. For example, when blower 4 outputs air at the first air pressure, blower 4 may output the air at the first air volume. When blower 4 outputs air at the second air pressure, the blower 4 may output the air at the second air volume.

As illustrated in Fig. 3, evaporator 5 is disposed in the middle of the flow of air (that is, the flow of air flowing inside the flow path) generated by blower 4. More specifically, evaporator 5 is disposed downstream of blower 4 in the first direction. Evaporator 5 has a heat exchange part which is a gap through which air flowing in case 20 passes. At a position where evaporator 5 is disposed, an outer edge of evaporator 5 (more specifically, a transverse sectional area of evaporator 5) and an inner edge of case 20 (more specifically, a transverse sectional area of case 20) are substantially same as each other and are in contact with each other.

Evaporator 5 in the first exemplary embodiment is connected to a refrigerant circulation flow path extending from engine room 16 (see Fig. 1) to device room 15 (see Fig. 1). A compressor, a condenser, a receiver, and an expansion valve are also connected to the refrigerant circulation flow path. When power is transmitted from the engine to the compressor, the compressor circulates the refrigerant in the circulation flow path. The refrigerant circulating in the circulation path vaporizes in evaporator 5. When the refrigerant is vaporized, that is, when evaporator 5 is operating, the refrigerant takes heat from the air passing through the gap (more specifically, the heat exchange part) of evaporator 5 as heat of vaporization, whereby the air is cooled. Evaporator 5 is controlled by controller 8.

As illustrated in Fig. 3, heater 6 is disposed downstream of evaporator 5 in the first direction. Heater 6 has a heat exchange part that is a gap through which air passes. Heater 6 is disposed such that, when air is flowing in the first direction, a part of the air that has passed through evaporator 5 passes through heater 6 (more specifically, the heat exchange part of heater 6). An outer edge of heater 6 (more specifically, a transverse sectional area of heater 6) is smaller than an inner edge of case 20 (more specifically, a transverse sectional area of case 20) at a position where heater 6 is disposed.

Heater 6 in the first exemplary embodiment is connected, together with a hot water valve, to a distributary channel for engine cooling water from engine room 16 (see Fig. 1) to device room 15 (see Fig. 1). The distributary channel is disposed such that heater 6 and the radiator are arranged in parallel to each other in a circulation flow path of the engine cooling water circulating between the engine and the radiator. When the hot water valve is opened while the engine cooling water is circulating in the circulation flow path, the cooling water (that is, hot water) heated by the engine flows into the distributary channel, and heater 6 is heated by the hot water. The air passes through the heat exchange part of heated heater 6, whereby the air is heated. Heater 6 (more specifically, hot water valve) is controlled by controller 8.

Heater valve 295 is disposed upstream of heater 6 in the first direction. The opening degree (that is, the degree of the opening of the valve) of heater valve 295 is controlled according to the control of controller 8. The amount of air to pass through heater 6 is adjusted according to the opening degree of heater valve 295.

Filter 7 is disposed upstream of blower 4 and downstream of internal air introduction port 24 and external air introduction port 25 in the first direction. At the position where filter 7 is disposed, the outer edge of the filter 7 (more specifically, a transverse sectional area of filter 7) and the inner edge of the case 20 (more specifically, a transverse sectional area of case 20) are substantially same as each other and are in contact with each other. That is, the air flowing from internal air introduction port 24 and external air introduction port 25 toward the inside of case 20 or the air flowing from the inside of case 20 toward internal air introduction port 24 and external air introduction port 25 passes through filter 7. Filter 7 purifies the air flow by removing dust and dirt included in the air flow passing through filter 7.

Functional substance generator 3 generates a functional substance. Functional substance generator 3 in the first exemplary embodiment includes a cooling unit including a Peltier element, a discharge electrode, a counter electrode, and a voltage application unit. In functional substance generator 3, the Peltier element is energized to cool the cooling unit and cool the discharge electrode. When the discharge electrode is cooled, dew condensation occurs at the discharge electrode. When the voltage application unit applies a high voltage between the discharge electrode and the counter electrode in a state where dew condensation has occurred at the discharge electrode, a Taylor cone is formed in the discharge electrode. When electric charges are concentrated at the tip of the Taylor cone and the field intensity at the tip of the Taylor cone has increased, the Coulomb force generated at the tip of the Taylor cone increases, and the Taylor cone grows. When the Taylor cone grows, and electric charges are concentrated at the tip of the Taylor cone to increase the density of charges, dew condensation water at the tip of the Taylor cone receives a large amount of energy (more specifically, the repulsive force of the high-density electric charges) and repeatedly splits and scatters (more specifically, Rayleigh splitting) beyond the surface tension to generate a large amount of charged fine water particles having a nanometer size. Functional substance generator 3 has a motor fan for easily releasing the generated functional substance to the outside thereof. Functional substance generator 3 operates under the control of controller 8.

As illustrated in Fig. 3, functional substance generator 3 in the first exemplary embodiment is disposed outside case 20. The functional substance generated by functional substance generator 3 is sent into case 20 through functional substance duct D0, case duct D1, and junction port 201. Since functional substance generator 3 is disposed outside case 20, case 20 can be downsized. In addition, since functional substance generator 3 is disposed outside case 20, maintenance and repair of functional substance generator 3 can be easily performed.

Functional substance generator 3 in the first exemplary embodiment is connected to air duct D3 via functional substance duct D0 and vehicle-interior duct D2. That is, the functional substance generated by functional substance generator 3 is sent into at least one of vehicle interior 10 and case 20 through functional substance duct D0, vehicle-interior duct D2, and air duct D3. For example, by controlling case valve 26, the vehicle-interior valve 27, and air duct valve 28, the functional substance generated by functional substance generator 3 can be selectively moved.

Operation unit 9 is provided on instrument panel 12 (see Fig. 2). Operation unit 9 includes, for example, a touch panel display. Operation unit 9 receives operations by a user of vehicle 1. In accordance with a user's operation, operation unit 9 receives operations such as on/off of an air conditioning operation of air conditioning system 2, switching between a cooling operation and a heating operation, temperature setting, air volume setting, on/off of functional substance generator 3, and selection of an operation mode, which will be described later.

Air conditioning system 2 includes, for example, a microcomputer including a processor and a memory. When the processor executes an appropriate program, the computer system functions as controller 8. That is, controller 8 is realized by a computer system including a processor and a memory. The program may be recorded in advance in the memory or may be provided through an electric telecommunication line such as the Internet or provided being recorded in a non-transitory recording medium such as a memory card.

Controller 8 performs drive control of air conditioning system 2. Controller 8 performs drive control of air conditioning system 2 based on a detection result provided from a detector such as a seat sensor, a human sensor, an infrared sensor, an odor sensor, a PM2.5 sensor, an ozone sensor, a temperature sensor, a humidity sensor, a speed sensor, an acceleration sensor, a gyro sensor, a CO₂ sensor, or a camera, or a control signal corresponding to the user's operation on operation unit 9. For example, controller 8 executes a plurality of operation modes based on the control signal. The plurality of operation modes includes an exposure mode (more specifically, normal mode), a drying mode, and a filling mode. That is, controller 8 is configured to execute the exposure mode, the drying mode, and the filling mode. The detector may be a component of air conditioning system 2 or a component different from air conditioning system 2 of vehicle 1.

The exposure mode is an operation mode in which evaporator 5 is exposed to the air flow containing the functional substance generated by functional substance generator 3. When air conditioning system 2 operates in the exposure mode, controller 8 operates blower 4 so that air is output from blower 4 at the first air pressure.

The drying mode is an operation mode for drying evaporator 5. When air conditioning system 2 operates in the drying mode, controller 8 operates blower 4 so that air is output from blower 4 at the second air pressure higher than the first air pressure. When evaporator 5 is operating, dew condensation may occur in evaporator 5 because of a temperature difference between air and evaporator 5. By drying evaporator 5 in the drying mode, an environment in which mold generation and growth are difficult can be created.

When air conditioning system 2 operates in the drying mode, controller 8 in the first exemplary embodiment operates heater 6 and operates blower 4 to generate a flow of air in the second direction, which is opposite to the first direction. When blower 4 generates the flow of air in the second direction, at least one of the plurality of air duct valves 28 is preferably open. Blower 4 is operated to generate the flow of air in the second direction, and heater 6 located upstream of evaporator 5 in the second direction is operated, whereby evaporator 5 can be exposed to the hot air, and evaporator 5 can be dried well. It is not essential that controller 8 controls blower 4 so that blower 4 generates the flow of air in the second direction.

The filling mode is an operation mode in which case 20 is filled with a functional substance to expose evaporator 5 to the functional substance. When air conditioning system 2 operates in the filling mode, controller 8 operates functional substance generator 3, stops blower 4, opens case valve 26, and closes air duct valve 28 to fill case 20 with the functional substance. By filling case 20 with the functional substance, the surface of evaporator 5 can be entirely exposed to the functional substance. In addition, by filling case 20 with the functional substance, bacteria inactivation can be performed not only on evaporator 5 but also on the surface in the case 20, blower 4, and filter 7.

After a lapse of a predetermined time from the start of execution of the filling mode, controller 8 may open at least one of the plurality of air duct valves 28, control introduction port valve 291 so that air flows to external-air duct D8, and operate blower 4 so as to generate a flow of air in the second direction. By generating the flow of air in the second direction, which is opposite to the first direction toward vehicle interior 10, and controlling the air to exit to the outside of the vehicle, the functional substance filled in case 20 can be discharged not into the vehicle interior 10 but to the outside of the vehicle. In particular, when functional substance generator 3 generates ozone, ozone filled in case 20 can be prevented from moving into vehicle interior 10.

### (3) Operation of air conditioning system

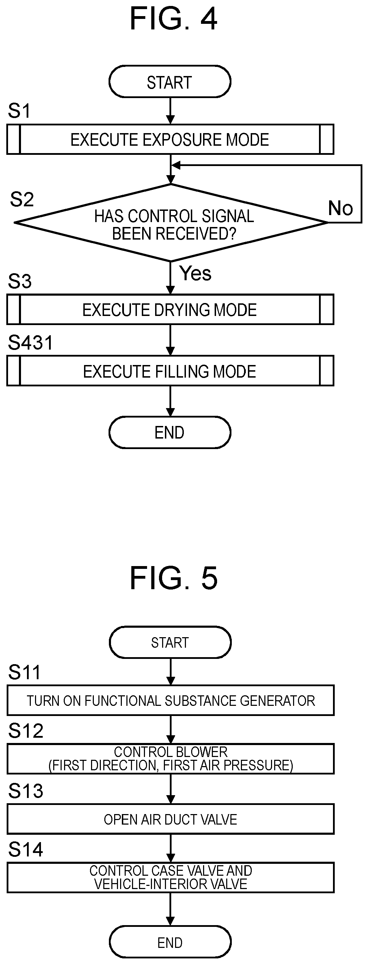

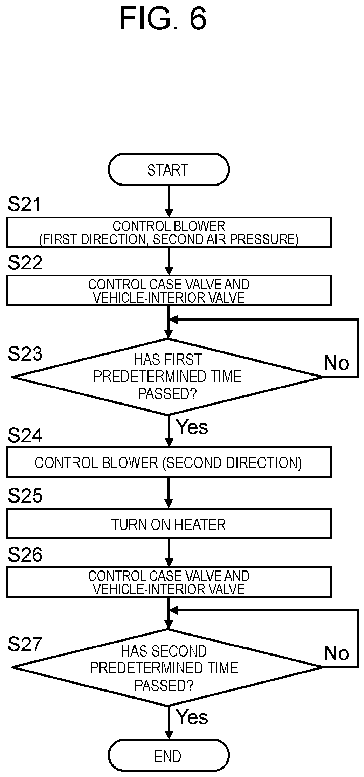

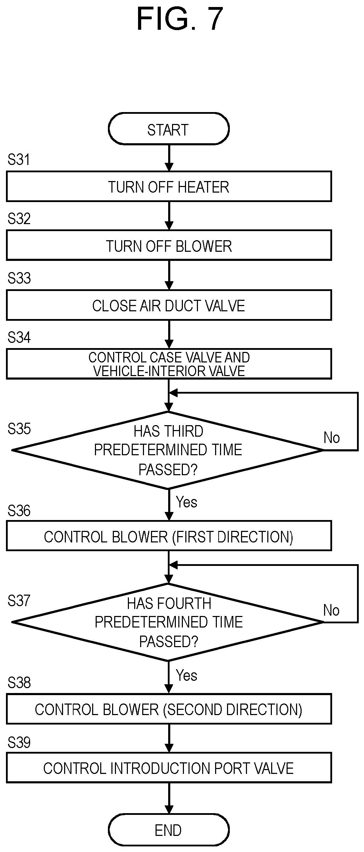

Next, the operation of air conditioning system 2 will be described with reference to Figs. 4 to 7. Fig. 4 is a flowchart illustrating an operation of air conditioning system 2 according to the first exemplary embodiment. Fig. 5 is a flowchart illustrating the operation in the exposure mode of air conditioning system 2 according to the first exemplary embodiment. Fig. 6 is a flowchart illustrating an operation in the drying mode of air conditioning system 2 according to the first exemplary embodiment. Fig. 7 is a flowchart illustrating an operation in the filling mode of air conditioning system 2 according to the first exemplary embodiment. A series of processing illustrated in the flowchart of Fig. 4 is processing for performing bacteria inactivation on evaporator 5, and is, for example, processing to be started when a user performs a predetermined operation on operation unit 9.

First, controller 8 executes the exposure mode (S1). Next, controller 8 determines whether a control signal has been received from operation unit 9 (S2). Here, the control signal is a signal transmitted from operation unit 9 to controller 8, for example, when operation unit 9 has received a predetermined operation from the user. If controller 8 has not received the control signal (S2: No), controller 8 repeats the processing in step S2 while executing the exposure mode, for example. On the other hand, if controller 8 has received the control signal (S2: Yes), controller 8 executes the drying mode (S3). After executing the drying mode, controller 8 executes the filling mode (S4) and ends the processing.

The flowchart illustrated in Fig. 4 is merely an example, and the order of the processing may be appropriately changed, or processing may be appropriately added or deleted. For example, controller 8 may execute only one of the drying mode and the filling mode after executing the exposure mode.

In this manner, controller 8 in the first exemplary embodiment executes at least one of the drying mode and the filling mode after executing the exposure mode. By further drying evaporator 5 or performing bacteria inactivation on evaporator 5 after performing bacteria inactivation on evaporator 5 in the exposure mode, bacteria inactivation on evaporator 5 can be more satisfactorily performed. For example, controller 8 may execute the exposure mode again after executing the filling mode.

Controller 8 in the first exemplary embodiment executes the exposure mode, the drying mode, and the filling mode in this order. Bacteria inactivation on evaporator 5 can be more satisfactorily performed by performing bacteria inactivation on evaporator 5 in the exposure mode, drying evaporator 5, and then, performing bacteria inactivation on evaporator 5 again.

Next, processing in the exposure mode (S1) will be described with reference to Fig. 5. When executing the exposure mode, controller 8 turns on functional substance generator 3 (S11). That is, when executing the exposure mode, controller 8 operates functional substance generator 3. Next, controller 8 controls blower 4 (S12). More specifically, controller 8 controls blower 4 so that blower 4 outputs air in the first direction at the first air pressure. Next, controller 8 opens air duct valve 28 (step S13).

Next, controller 8 controls case valve 26 and the vehicle-interior valve 27 (S14), and ends the exposure mode. More specifically, controller 8 controls case valve 26 and vehicle-interior valve 27 to open case valve 26. When case valve 26 is opened, the functional substance generated by functional substance generator 3 moves into case 20. The functional substance which has moved into case 20 passes through evaporator 5 together with the air flow in the first direction generated by blower 4, and moves into vehicle interior 10.

Next, processing in the drying mode (S3) will be described with reference to Fig. 6. When executing the drying mode, controller 8 controls blower 4 (S21). More specifically, controller 8 controls blower 4 so that blower 4 outputs air in the first direction at the second air pressure.

Next, controller 8 controls case valve 26 and vehicle-interior valve 27 (S22). More specifically, controller 8 controls case valve 26 and vehicle-interior valve 27 so as to close case valve 26 and open vehicle-interior valve 27. When case valve 26 is closed and vehicle-interior valve 27 is opened, the functional substance generated by functional substance generator 3 can be moved into vehicle interior 10 while evaporator 5 is dried. Controller 8 may control case valve 26 and vehicle-interior valve 27 so as to open case valve 26 and close vehicle-interior valve 27 so that the functional substance moves into case 20.

Next, controller 8 determines whether a predetermined time (hereinafter, referred to as "first predetermined time") has elapsed since the execution of the drying mode (S23). The first predetermined time is appropriately set according to the air pressure of the air output from blower 4 and the size of evaporator 5, or in accordance with the user's operation on operation unit 9. Instead of determining whether the first predetermined time has elapsed since the execution of the drying mode, controller 8 may determine whether the detection result provided by the detector is a predetermined result, or determine whether a predetermined operation has been performed on operation unit 9. If the first predetermined time has not elapsed since the execution of the drying mode (S23: No), controller 8 repeats the processing of step S23. If the first predetermined time has elapsed since the execution of the drying mode (S23: Yes), controller 8 controls blower 4 (S24). More specifically, controller 8 controls blower 4 to output air in the second direction. The air pressure of the air output from blower 4 may be either the first air pressure or the second air pressure, or may be a third air pressure different from the first air pressure or the second air pressure.

Next, controller 8 turns on heater 6 (S25). That is, controller 8 operates heater 6. Controller 8 then controls case valve 26 and vehicle-interior valve 27 (S26). More specifically, controller 8 controls case valve 26 and vehicle-interior valve 27 so as to open case valve 26 and close vehicle-interior valve 27.

Next, controller 8 determines whether a predetermined time (hereinafter, referred to as "second predetermined time") has elapsed since the execution of the processing of step S26 (S26). The second predetermined time is appropriately set according to the air pressure of the air output from blower 4, the size of evaporator 5 and the temperature of heater 6, or in accordance with the user's operation on operation unit 9. Instead of determining whether the second predetermined time has elapsed since the execution of the processing of step S26, controller 8 may determine whether the detection result provided by the detector is a predetermined result, or determine whether a predetermined operation has been performed on operation unit 9. If the second predetermined time has not elapsed since the execution of the processing of step S26 (S26: No), controller 8 repeats the processing of step S26. If the second predetermined time has elapsed since the execution of the processing of step S26 (S26: Yes), controller 8 ends the drying mode.

Next, processing in the filling mode (S4) will be described with reference to Fig. 7. When executing the filling mode, controller 8 turns off heater 6 (S31), turns off blower 4 (S32), and closes air duct valve 28 (S33).

Next, controller 8 controls case valve 26 and vehicle-interior valve 27 (S34). More specifically, controller 8 controls case valve 26 and vehicle-interior valve 27 so as to open case valve 26 and close vehicle-interior valve 27. When controller 8 turns off blower 4 and controls each valve, case 20 is filled with the functional substance.

Next, controller 8 determines whether a predetermined time (hereinafter, referred to as "third predetermined time") has elapsed since the execution of the processing of step S34 (S35). The third predetermined time is appropriately set according to the size of case 20, or in accordance with the user's operation on operation unit 9. Instead of determining whether the third predetermined time has elapsed since the execution of the processing of step S34, controller 8 may determine whether the detection result provided by the detector is a predetermined result, or determine whether a predetermined operation has been performed on operation unit 9. If the third predetermined time has not elapsed since the execution of the processing of step S34 (S35: No), controller 8 repeats the processing of step S35. If the third predetermined time has elapsed since the execution of the processing of step S34 (S35: Yes), controller 8 controls blower 4 (S36). More specifically, controller 8 controls blower 4 to output air in the first direction.

Next, controller 8 determines whether a predetermined time (hereinafter, referred to as "fourth predetermined time") has elapsed since the execution of the processing of step S36 (S37). The fourth predetermined time is appropriately set according to the air pressure of the air output from blower 4 and the size of evaporator 5, or in accordance with the user's operation on operation unit 9. Instead of determining whether the fourth predetermined time has elapsed since the execution of the processing of step S36, controller 8 may determine whether the detection result provided by the detector is a predetermined result, or determine whether a predetermined operation has been performed on operation unit 9. If the fourth predetermined time has not elapsed since the execution of the processing of step S36 (S37: No), controller 8 repeats the processing of step S37. If the fourth predetermined time has elapsed since the execution of the processing of step S36 (S37: Yes), controller 8 controls blower 4 (S38). More specifically, controller 8 controls blower 4 to output air in the second direction.

Next, controller 8 controls introduction port valve 291 (S39) to end the filling mode. Specifically, controller 8 controls introduction port valve 291 such that air flows to external-air duct D8. By generating flow of air from blower 4 toward external air introduction port 25, bacteria inactivation on filter 7 can be performed by exposing filter 7 to the functional substance. When the functional substance is ozone, the ozone can be moved outside the vehicle.

The flowcharts illustrated in Figs. 5 to 7 are merely an example, and the order of the processing may be appropriately changed, or processing may be appropriately added or deleted.

For example, in the processing of step S38 in Fig. 7, in addition to the control of blower 4, controller 8 may perform control to close case valve 26 and open vehicle-interior valve 27. That is, in the filling mode, controller 8 opens vehicle-interior valve 27 and operates blower 4 to generate flow of air in the second direction, which is opposite to the first direction. By generating the flow of air from blower 4 toward external air introduction port 25, the functional substance can be sent into vehicle interior 10 while performing bacteria inactivation on filter 7.

### (4) Modifications

Hereinafter, modifications of the first exemplary embodiment will be listed.

### (4.1) First modification

Fig. 8 is a schematic view illustrating a configuration of air conditioning system 2 according to a first modification of the first exemplary embodiment. As illustrated in Fig. 8, junction port 201 may be formed between blower 4 and filter 7. In other words, junction port 201 may be formed upstream of blower 4 in the first direction. Similarly to air conditioning system 2 according to the first exemplary embodiment, air conditioning system 2 according to the first modification can expose evaporator 5 to the functional substance generated by functional substance generator 3, for example, in the exposure mode.

### (4.2) Second modification

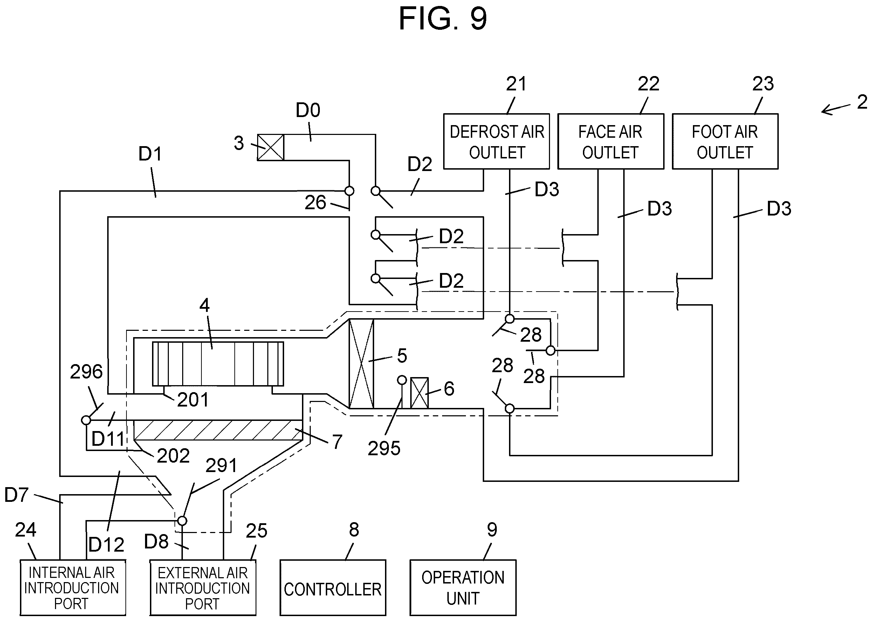

Fig. 9 is a schematic view illustrating a configuration of air conditioning system 2 according to a second modification of the first exemplary embodiment. As illustrated in Fig. 9, junction port 201 may be formed between blower 4 and filter 7, and junction port 202 different from junction port 201 may be formed upstream of filter 7 in the first direction. One end of case duct D1 is connected to functional substance duct D0, and the other end of case duct D1 is branched into first case duct D11 and second case duct D12. First case duct D11 is connected to junction port 201, and second case duct D12 is connected to junction port 202.

In air conditioning system 2 according to the second modification, the destination of the functional substance generated by functional substance generator 3 can be selected from between blower 4 and filter 7 and the upstream of filter 7 in the first direction. For example, in the filling mode, controller 8 turns on functional substance generator 3, turns off blower 4, and controls case valve 26, vehicle-interior valve 27, and valve 296. Thus, filter 7 and internal air introduction port 24 and external air introduction port 25 can be filled with the functional substance.

### (4.3) Third modification

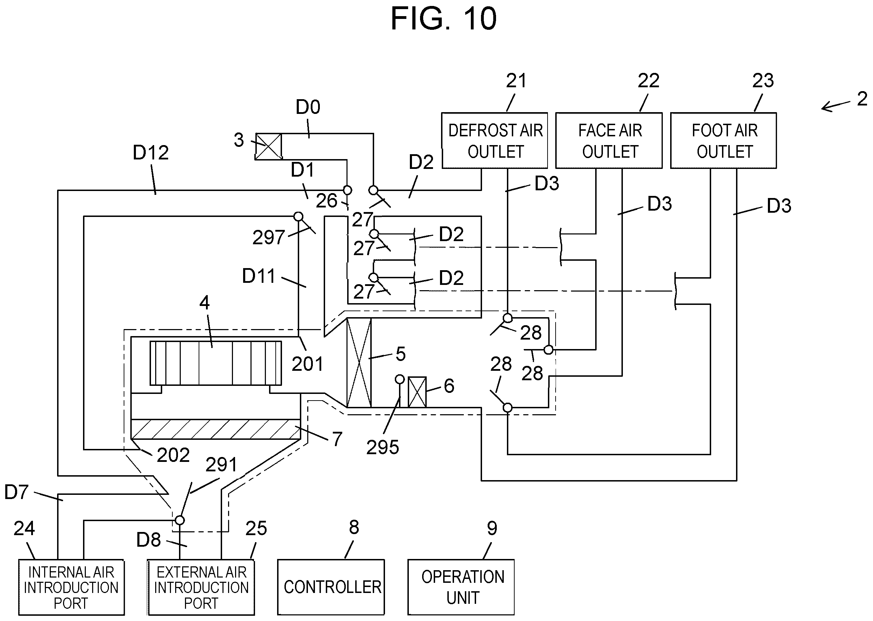

Fig. 10 is a schematic view illustrating a configuration of air conditioning system 2 according to a third modification of the first exemplary embodiment. As illustrated in Fig. 10, junction port 201 may be formed between blower 4 and evaporator 5, and junction port 202 may be formed upstream of filter 7 in the first direction. First case duct D11 is connected to junction port 201, and second case duct D12 is connected to junction port 202.

Valve 297 is a valve that can plug (that is, close) one of first case duct D11 and second case duct D12.

In air conditioning system 2 according to the third modification, the destination of the functional substance generated by functional substance generator 3 can be selected from between blower 4 and evaporator 5 and the upstream of filter 7 in the first direction.

### (4.4) Fourth modification

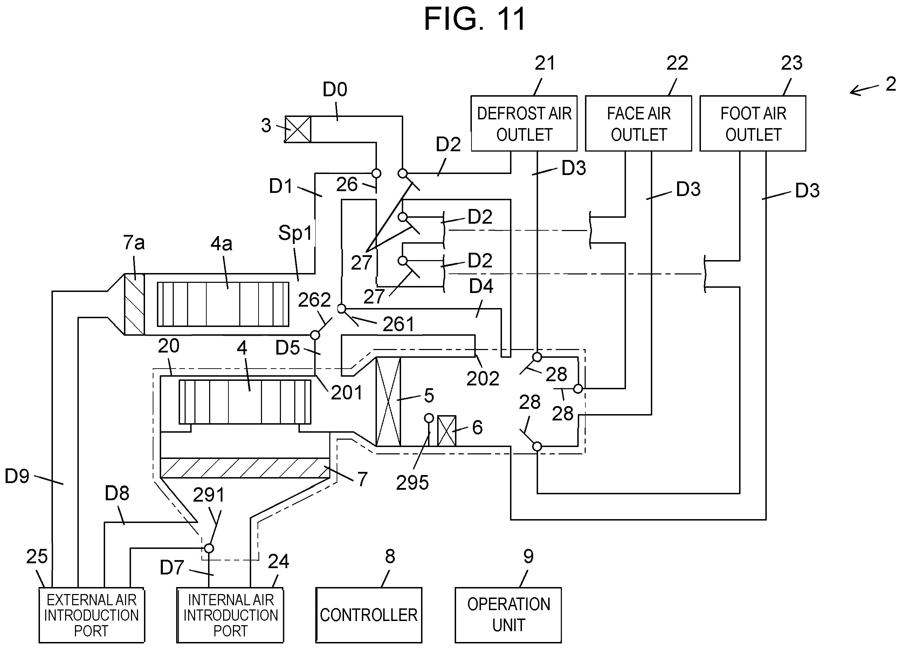

Fig. 11 is a schematic view illustrating a configuration of air conditioning system 2 according to a fourth modification of the first exemplary embodiment. As illustrated in Fig. 11, air conditioning system 2 may further include second direction blower 4a that generates a flow of air in the second direction, which is opposite to the first direction, filter 7a, and duct D9 connecting filter 7a to external air introduction port 25. Second direction blower 4a of the fourth modification generates a flow of air in the direction from external air introduction port 25 toward filter 7a via duct D9 and the direction from filter 7a toward second direction blower 4a. Blower 4 (that is, the first direction blower) of the fourth modification can generate, for example, only a flow of air in the first direction.

Second direction blower 4a and filter 7a are disposed outside case 20. Space Sp1 in which second direction blower 4a is disposed is connected to the other end of case duct D1, one end of duct D4, and one end of duct D5. Space Sp1 in which second direction blower 4a is disposed is connected to duct D9 via filter 7a. However, second direction blower 4a and filter 7a may be disposed inside case 20.

Junction port 201 is formed between blower 4 and evaporator 5. Junction port 202 is formed at a position downstream of evaporator 5 and upstream of air duct D3 in the first direction.

One end of case duct D1 is connected to functional substance duct D0, and the other end of case duct D1 is connected to space Sp1 in which second direction blower 4a is disposed. One end of duct D4 is connected to space Sp1, and the other end of duct D4 is connected to junction port 202. Junction port 202 of the fourth modification is provided upstream of heater 6 in the second direction. One end of duct D5 is connected to space Sp1, and the other end of duct D5 is connected to junction port 201.

Valve 261 opens and closes the flow path of duct D4, and valve 262 opens and closes the flow path of duct D5.

Air conditioning system 2 (more specifically, controller 8) of the fourth modification performs the following processing instead of steps S24 to S26 in Fig. 6 in the drying mode.

Controller 8 turns off blower 4, turns on second direction blower 4a, opens valve 261, and closes valve 262. With the operation of second direction blower 4a, a flow of air passing through external air introduction port 25, duct D9, filter 7a, space Sp1, duct D4, heater 6, evaporator 5, blower 4, filter 7, and internal air introduction port 24 or external air introduction port 25 in this order is formed. That is, with the operation of second direction blower 4a, a flow of air in the second direction from vehicle interior 10 toward blower 4 and from blower 4 toward internal air introduction port 24 or external air introduction port 25 is formed. Controller 8 turns on heater 6. Controller 8 closes case valve 26 and opens vehicle-interior valve 27. However, controller 8 may open case valve 26 and close vehicle-interior valve 27.

That is, controller 8 in the fourth modification is configured to execute a drying mode in which heater 6 is operated, second direction blower 4a is operated without operating blower 4, and evaporator 5 is dried. With controller 8 of the fourth modification, even when blower 4 is not configured to generate a flow air in the reverse direction (that is, the second direction), evaporator 5 can be exposed to hot air, and evaporator 5 can be dried well.

In addition, controller 8 in the fourth modification performs the following processing instead of step S38 of Fig. 7 in the filling mode.

Controller 8 generates a flow of air in the second direction by turning off blower 4, turning on second direction blower 4a, opening valve 261, and closing valve 262. By generating a flow of air in the second direction, the functional substance filled in case 20 can be moved to the outside of the vehicle via duct D9 and external air introduction port 25.

Duct D9 may be a duct connecting internal air introduction port 24 to space Sp1 via filter 7a.

### (4.5) Fifth modification

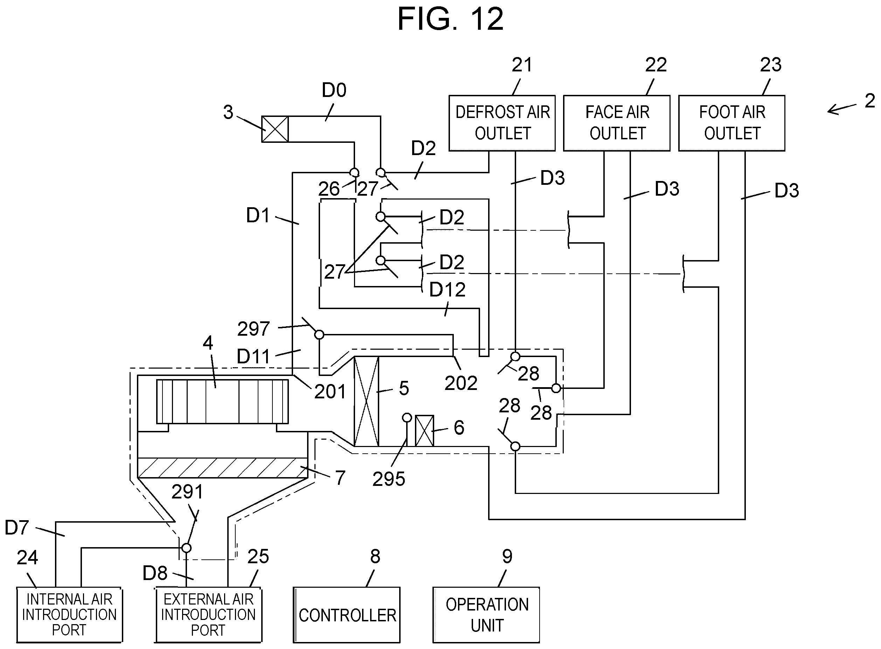

Fig. 12 is a schematic view illustrating a configuration of air conditioning system 2 according to a fifth modification of the first exemplary embodiment. As illustrated in Fig. 12, junction port 201 may be formed between blower 4 and evaporator 5, and junction port 202 may be formed downstream of evaporator 5 and upstream of air duct D3 in the first direction. To distinguish junction port 201 and junction port 202 from each other, junction port 201 and junction port 202 may be referred to as first junction port 201 and second junction port 202, respectively. First case duct D11 is connected to junction port 201, and second case duct D12 is connected to junction port 202. Junction port 202 is disposed upstream of heater 6 in the second direction.

In air conditioning system 2 according to the fifth modification, the destination of the functional substance generated by functional substance generator 3 can be selected from between blower 4 and evaporator 5 and the downstream of evaporator 5 in the first direction.

### (4.6) Other modifications

The control of functional substance generator 3, blower 4, heater 6, and each valve described in the first exemplary embodiment and the first modification to the fifth modification may be appropriately changed without departing from the purpose of each operation mode. The control of each valve includes not only the control to completely open and the control to completely close, but also the control to open or close a part of a flow path by controlling the opening/closing angle of each valve.

In the first exemplary embodiment, air conditioning system 2 includes a plurality of vehicle-interior ducts D2. In the first exemplary embodiment, three vehicle-interior ducts D2 are provided. However, air conditioning system 2 may include at least one of the plurality of vehicle-interior ducts D2 or may include no vehicle-interior duct D2.

### (Second exemplary embodiment)

Next, air conditioning system 2 according to a second exemplary embodiment will be described.

### (1) Configuration

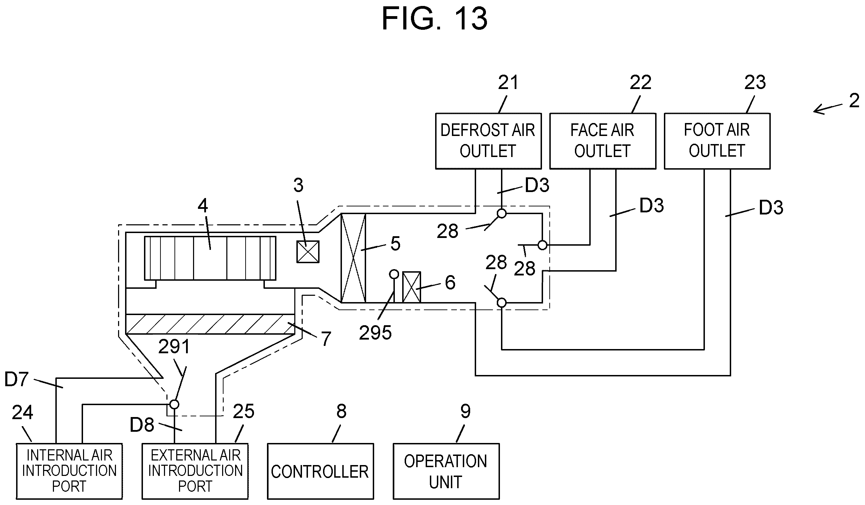

Fig. 13 is a schematic view illustrating a configuration of air conditioning system 2 according to the second exemplary embodiment. As illustrated in Fig. 13, in air conditioning system 2 according to the second exemplary embodiment, functional substance generator 3 is disposed in case 20. More specifically, functional substance generator 3 is disposed between blower 4 and evaporator 5.

Since functional substance generator 3 is disposed in case 20, it is not necessary to move the functional substance generated by functional substance generator 3 from the outside of case 20 into case 20, and it is possible to suppress reduction of the functional substance during the movement of the functional substance. Functional substance generator 3 is disposed upstream of evaporator 5 in the first direction. In the same manner as in air conditioning system 2 according to the first exemplary embodiment, the functional substance flows toward evaporator 5 and passes through evaporator 5 during the operation of blower 4 in air conditioning system 2 according to the second exemplary embodiment, and thus evaporator 5 is easily exposed to the functional substance.

In air conditioning system 2 according to the second exemplary embodiment, since functional substance generator 3 is disposed between blower 4 and evaporator 5, evaporator 5 can be exposed to the functional substance more satisfactorily as compared with, for example, the case where functional substance generator 3 is disposed upstream of blower 4 in the first direction. According to air conditioning system 2 according to the second exemplary embodiment, entire case 20 can be filled with the functional substance in the filling mode.

### (2) Operation of air conditioning system

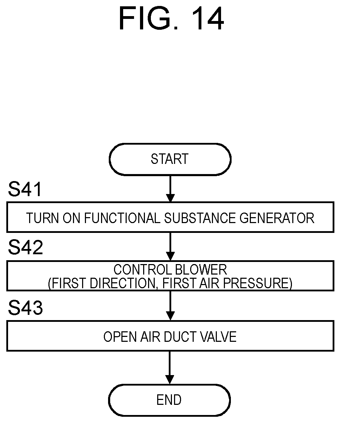

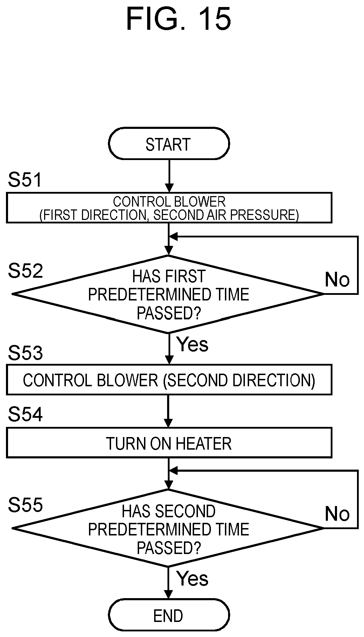

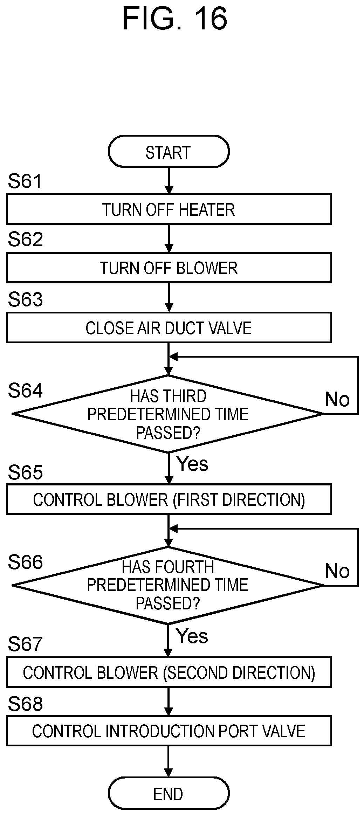

Next, an operation of air conditioning system 2 according to the second exemplary embodiment will be described with reference to Figs. 4 and 14 to 16. Fig. 14 is a flowchart illustrating the operation in the exposure mode of air conditioning system 2 according to the second exemplary embodiment. Fig. 15 is a flowchart illustrating an operation in the drying mode of air conditioning system 2 according to the second exemplary embodiment. Fig. 16 is a flowchart illustrating an operation in the filling mode of air conditioning system 2 according to the second exemplary embodiment. In the same manner as in the case of air conditioning system 2 according to the first exemplary embodiment, air conditioning system 2 according to the second exemplary embodiment starts the processing illustrated in Fig. 4 when, for example, a user performs a predetermined operation on operation unit 9.

First, processing in the exposure mode (S1) will be described with reference to Fig. 14. When executing the exposure mode, air conditioning system 2 (more specifically, controller 8) turns on functional substance generator 3 (S41). That is, when executing the exposure mode, air conditioning system 2 (more specifically, controller 8) operates functional substance generator 3. Next, controller 8 controls blower 4 (S42). More specifically, controller 8 controls blower 4 so that blower 4 outputs air in the first direction at the first air pressure. Next, controller 8 opens air duct valve 28 (S43) and ends the exposure mode.

Next, processing in the drying mode (S3) will be described with reference to Fig. 15. When executing the drying mode, controller 8 controls blower 4 (S51). More specifically, controller 8 controls blower 4 so that blower 4 outputs air in the first direction at the second air pressure.

Next, controller 8 determines whether a predetermined time (hereinafter, referred to as "first predetermined time") has elapsed since the execution of the drying mode (S52). Instead of determining whether the first predetermined time has elapsed since the execution of the drying mode, controller 8 may determine whether the detection result provided by the detector is a predetermined result, or determine whether a predetermined operation has been performed on operation unit 9. If the first predetermined time has not elapsed since the execution of the drying mode (S52: No), controller 8 repeats the processing of step S52. If the first predetermined time has elapsed since the execution of the drying mode (S52: Yes), controller 8 controls blower 4 (S53). More specifically, controller 8 controls blower 4 to output air in the second direction.

Next, controller 8 turns on heater 6 (S54). That is, controller 8 operates heater 6. Then, controller 8 determines whether a predetermined time (hereinafter, referred to as "second predetermined time") has elapsed since the execution of the processing of step S54 (S55). Instead of determining whether the second predetermined time has elapsed since the execution of the processing of step S54, controller 8 may determine whether the detection result provided by the detector is a predetermined result, or determine whether a predetermined operation has been performed on operation unit 9. If the second predetermined time has not elapsed since the execution of the processing of step S54 (S55: No), controller 8 repeats the processing of step S55. If the second predetermined time has elapsed since the execution of the processing of step S54 (S55: Yes), controller 8 ends the drying mode.

Next, processing in the filling mode (S4) will be described with reference to Fig. 16. When executing the filling mode, controller 8 turns off heater 6 (S61), turns off blower 4 (S62), and closes air duct valve 28 (S63).

Next, controller 8 determines whether a predetermined time (hereinafter, referred to as "third predetermined time") has elapsed since the execution of the processing of step S63 (S64). Instead of determining whether the third predetermined time has elapsed since the execution of the processing of step S63, controller 8 may determine whether the detection result provided by the detector is a predetermined result, or determine whether a predetermined operation has been performed on operation unit 9. If the third predetermined time has not elapsed since the execution of the processing of step S63 (S64: No), controller 8 repeats the processing of step S64. If the third predetermined time has elapsed since the execution of the processing of step S63 (S64: Yes), controller 8 controls blower 4 (S65). More specifically, controller 8 controls blower 4 to output air in the first direction.

Next, controller 8 determines whether a predetermined time (hereinafter, referred to as "fourth predetermined time") has elapsed since the execution of the processing of step S65 (S66). Instead of determining whether the fourth predetermined time has elapsed since the execution of the processing of step S65, controller 8 may determine whether the detection result provided by the detector is a predetermined result, or determine whether a predetermined operation has been performed on operation unit 9. If the fourth predetermined time has not elapsed since the execution of the processing of step S65 (S66: No), controller 8 repeats the processing of step S66. If the fourth predetermined time has elapsed since the execution of the processing of step S65 (S66: Yes), controller 8 controls blower 4 (S67). More specifically, controller 8 controls blower 4 to output air in the second direction.

Next, controller 8 controls introduction port valve 291 (S68) and ends the filling mode. Specifically, controller 8 controls introduction port valve 291 such that air flows to external-air duct D8.

The flowcharts illustrated in Figs. 14 to 16 are merely an example, and the order of the processing may be appropriately changed, or processing may be appropriately added or deleted.

### (3) Modifications

Hereinafter, modifications of the second exemplary embodiment will be listed.

### (3.1) First modification

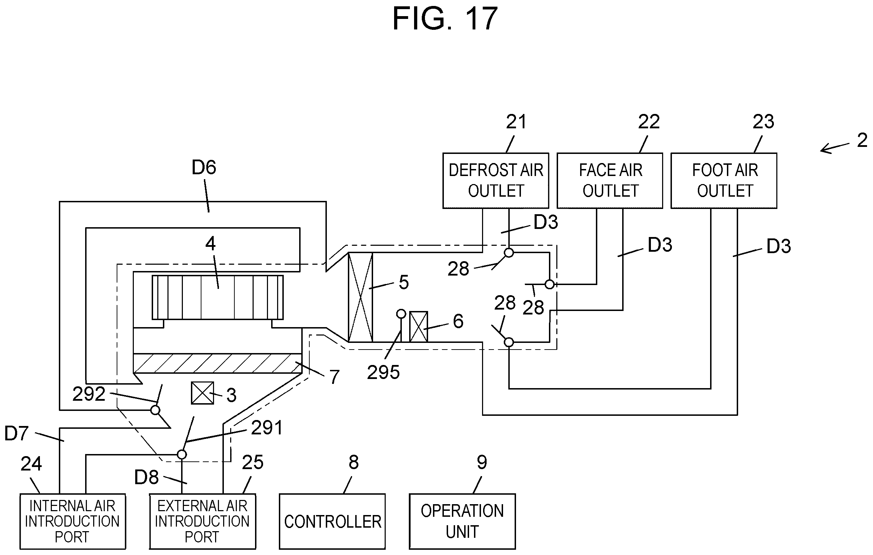

Fig. 17 is a schematic view illustrating a configuration of air conditioning system 2 according to a first modification of the second exemplary embodiment. As illustrated in Fig. 17, functional substance generator 3 may be disposed upstream of filter 7 in the first direction. Since functional substance generator 3 is disposed upstream of filter 7 in the first direction, bacteria inactivation can be performed on filter 7 when blower 4 is generating a flow of air in the first direction.

Air conditioning system 2 according to the first modification includes bypass duct D6. One end of bypass duct D6 is connected to a space between blower 4 and evaporator 5 in case 20. The other end of bypass duct D6 is connected to a space between filter 7 and internal air introduction port 24 and external air introduction port 25 in case 20. Valve 292 is provided on the other end side of bypass duct D6 and opens and closes the flow path of bypass duct D6.

For example, in the filling mode, the functional substance can be filled between blower 4 and evaporator 5 by opening valve 292. That is, in air conditioning system 2 according to the first modification, the place to be filled with the functional substance in the filling mode can be selected from between blower 4 and evaporator 5 and the upstream of filter 7 in the first direction.

### (3.2) Second modification

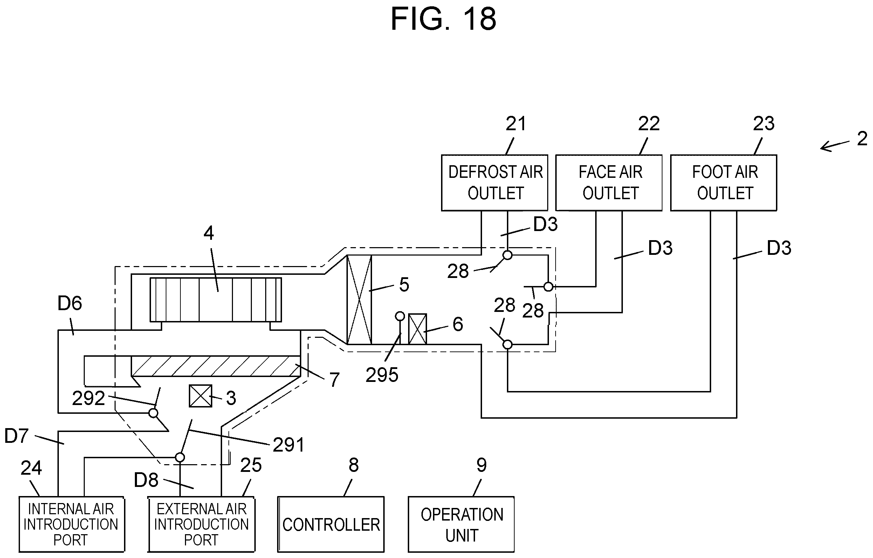

Fig. 18 is a schematic view illustrating a configuration of air conditioning system 2 according to a second modification of the second exemplary embodiment. As illustrated in Fig. 18, one end of bypass duct D6 may be connected to a space between blower 4 and filter 7 in case 20.

For example, in the filling mode, the functional substance can be filled between blower 4 and filter 7 by opening valve 292. That is, in air conditioning system 2 according to the second modification, the place to be filled with the functional substance in the filling mode can be selected from between blower 4 and filter 7 and the upstream of filter 7 in the first direction.

### (3.3) Third modification

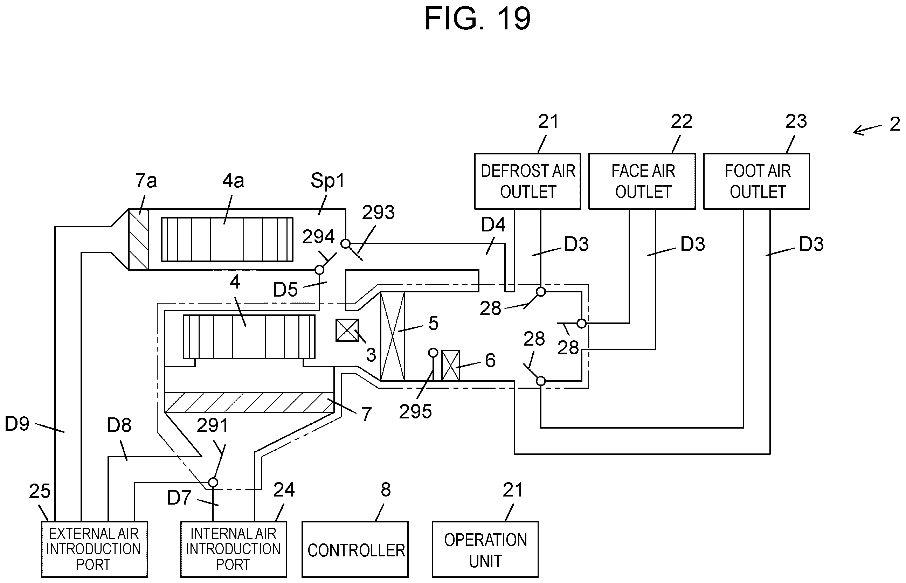

Fig. 19 is a schematic view illustrating a configuration of air conditioning system 2 according to a third modification of the second exemplary embodiment. As illustrated in Fig. 19, air conditioning system 2 may further include second direction blower 4a that generates a flow of air in the second direction, which is opposite to the first direction, filter 7a, and duct D9 connecting filter 7a and external air introduction port 25. Second direction blower 4a of the fourth modification generates a flow of air in the direction from external air introduction port 25 toward filter 7a via duct D9 and the direction from filter 7a toward second direction blower 4a. Blower 4 (that is, the first direction blower) of the fourth modification can generate, for example, only a flow of air in the first direction.

Second direction blower 4a and filter 7a are disposed outside case 20. Space Sp1 in which second direction blower 4a is disposed is connected to one end of duct D4 and one end of duct D5. Space Sp1 in which second direction blower 4a is disposed is connected to duct D9 via filter 7a. However, second direction blower 4a and filter 7a may be disposed inside case 20.

Functional substance generator 3 is disposed between blower 4 and evaporator 5.

One end of duct D4 is connected to space Sp1. The other end of duct D4 is connected to a space between evaporator 5 and air duct D3 in case 20, the space being disposed upstream of heater 6 in the second direction. One end of duct D5 is connected to space Sp1. The other end of duct D5 is connected to a space between blower 4 and evaporator 5 in case 20.

Valve 293 opens and closes the flow path of duct D4, and valve 294 opens and closes the flow path of duct D5.

Air conditioning system 2 (more specifically, controller 8) according to the third modification performs the following processing instead of steps S53 and S54 in Fig. 15 in the drying mode.

Controller 8 turns off blower 4, turns on second direction blower 4a, opens valve 293, and closes valve 294. With the operation of second direction blower 4a, a flow air passing through external air introduction port 25, duct D9, filter 7a, space Sp1, duct D4, heater 6, evaporator 5, the functional substance generator, blower 4, filter 7, and internal air introduction port 24 or external air introduction port 25 in this order is formed. That is, with the operation of second direction blower 4a, a flow of air in the second direction from vehicle interior 10 toward blower 4 and from blower 4 toward internal air introduction port 24 or external air introduction port 25 is formed. Controller 8 turns on heater 6. Controller 8 closes case valve 26 and opens vehicle-interior valve 27.

In addition, controller 8 of the third modification performs the following processing instead of step S67 of Fig. 16 in the filling mode.

Controller 8 generates a flow of air in the second direction by turning off blower 4, turning on second direction blower 4a, opening valve 293, and closing valve 294.

Duct D9 may be a duct connecting internal air introduction port 24 to space Sp1 via filter 7a.

### (3.4) Other modifications

The control of functional substance generator 3, blower 4, heater 6, and each valve described in the second exemplary embodiment and the first modification to the third modification may be appropriately changed without departing from the purpose of each operation mode. The control of each valve includes not only the control to completely open and the control to completely close, but also the control to open or close a part of a flow path by controlling the opening/closing angle of each valve.

### (Conclusions)

As described above, air conditioning system (2) according to a first aspect includes blower (4), evaporator (5), case (20), and functional substance generator (3). Blower (4) generates a flow of air toward vehicle interior (10). Evaporator (5) is disposed in the middle of a flow path of air and exchanges heat of air. Case (20) accommodates blower (4) and evaporator (5). Functional substance generator (3) generates a functional substance. Functional substance generator (3) is disposed upstream of evaporator (5) in a first direction which is a flow direction of the air toward vehicle interior (10).

In this aspect, since the functional substance flows toward evaporator (5) and passes through evaporator (5) at the time of the operation of blower (4), evaporator (5) is easily exposed to the functional substance. For example, even when evaporator (5) is large, since the functional substance passes through evaporator (5), the surface of evaporator (5) can be exposed to the functional substance as a whole. That is, bacteria inactivation on evaporator (5) can be performed more satisfactorily. In addition, bacteria inactivation on evaporator (5) can be performed at the time of the operation of blower (4), that is, during cooling operation or heating operation of air conditioning system (2).

Air conditioning system (2) according to a second aspect further includes case duct (D1) in the first aspect. Case duct (D1) connects case (20) to functional substance generator (3). Case (20) includes junction port (201) located upstream of evaporator (5) in the first direction. Functional substance generator (3) is disposed outside case (20). The functional substance is sent into case (20) through case duct (D1) and junction port (201).

In this aspect, since functional substance generator (3) is disposed outside case (20), case (20) can be downsized. In addition, since functional substance generator (3) is disposed outside case (20), maintenance and repair of functional substance generator (3) can be easily performed.

Air conditioning system (2) according to a third aspect further includes, in the second aspect, air duct (D3) and vehicle-interior duct (D2). Air duct (D3) is disposed downstream of evaporator (5) in the first direction and connects the inside of vehicle interior (10) and case (20). Vehicle-interior duct (D2) connects air duct (D3) and functional substance generator (3). The functional substance is sent into at least one of vehicle interior (10) and case (20) through vehicle-interior duct (D2).

In this aspect, the functional substance generated by functional substance generator (3) can be selectively moved by controlling a valve provided in case (20) or the duct, for example.

In air conditioning system (2) according to a fourth aspect, in the first aspect, functional substance generator (3) is disposed in case (20).

In this aspect, it is not necessary to move the functional substance generated by functional substance generator (3) from the outside of case (20) into case (20), and it is possible to suppress reduction of the functional substance during the movement of the functional substance.

In air conditioning system (2) according to a fifth aspect, in the fourth aspect, functional substance generator (3) is disposed between blower (4) and evaporator (5).

In this aspect, for example, evaporator (5) can be exposed to the functional substance more satisfactorily as compared with the case where functional substance generator (3) is disposed upstream of blower (4) in the first direction.

Air conditioning system (2) according to a sixth aspect further includes controller (8) in any one of the first to fifth aspects. Controller (8) performs drive control of air conditioning system (2). Controller (8) is configured to execute an exposure mode of operating blower (4) to output the air from blower (4) at a first air pressure and exposing evaporator (5) to the functional substance. Controller (8) is configured to execute a drying mode of operating blower (4) to output the air from blower (4) at a second air pressure higher than the first air pressure and drying evaporator (5).

In this aspect, it is possible to create an environment in which mold generation and growth are difficult.

Air conditioning system (2) according to a seventh aspect further includes, in any one of the first to sixth aspects, air duct (D3), air duct valve (28), and controller (8). Air duct (D3) is disposed downstream of evaporator (5) in the first direction and connects the inside of vehicle interior (10) and case (20). Air duct valve (28) opens and closes a flow path of air duct (D3). Controller (8) performs drive control of air conditioning system (2). Controller (8) is configured to execute a filling mode of closing air duct valve (28) and filling case (20) with the functional substance.

In this aspect, by filling case (20) with the functional substance, a surface of evaporator (5) can be exposed to the functional substance as a whole. In addition, bacteria inactivation can be performed on not only evaporator (5) but also the surface in case (20), blower (4), and filter (7).

In air conditioning system (2) according to an eighth aspect, in the seventh aspect, controller (8) operates blower (4) to generate a flow of the air in a second direction, which is opposite to the first direction after a lapse of a predetermined time from start of execution of the filling mode.

In this aspect, by generating the flow of air in the second direction, which is opposite to the first direction toward the vehicle interior (10), the functional substance filled in case (20) can be discharged not into vehicle interior (10) but to the outside of vehicle, for example. In particular, when functional substance generator (3) generates ozone, the ozone filled in case (20) can be prevented from moving into vehicle interior (10).

Air conditioning system (2) according to a ninth aspect further includes, in any one of the first to fifth aspects, heater (6) and controller (8). Heater (6) is disposed downstream of evaporator (5) in the first direction. Controller (8) performs drive control of air conditioning system (2). Controller (8) is configured to execute a drying mode of operating heater (6), operating blower (4) to generate a flow of air in a second direction, which is opposite to the first direction, and drying evaporator (5).

In this aspect, evaporator (5) can be exposed to hot air, and evaporator (5) can be dried well.

Air conditioning system (2) according to a tenth aspect further includes, in any one of the first to fifth aspects, controller (8), heater (6), and second direction blower (4a). Controller (8) performs drive control of air conditioning system (2). Heater (6) is disposed downstream of evaporator (5) in the first direction. Second direction blower (4a) generates a flow of air in a second direction, which is opposite to the first direction. Controller (8) is configured to execute a drying mode of operating heater (6), operating second direction blower (4a) without operating blower (4), and drying evaporator (5).

In this aspect, even when blower (4) is not configured to generate a flow of air in the reverse direction (namely, the second direction), evaporator (5) can be exposed to hot air to dry evaporator (5) well.

Air conditioning system (2) according to an eleventh aspect further includes, in the eighth aspect, air duct (D3), vehicle-interior duct (D2), and vehicle-interior valve (27). Air duct (D3) is disposed downstream of evaporator (5) in the first direction and connects the inside of vehicle interior (10) to case (20). Vehicle-interior duct (D2) connects air duct (D3) to functional substance generator (3). Vehicle-interior valve (27) opens and closes a flow path of vehicle-interior duct (D2). Controller (8) opens vehicle-interior valve (27) and operates blower (4) to generate a flow of air in the second direction, which is opposite to the first direction.

According to this aspect, by generating the flow of air in the second direction, the functional substance can be sent into vehicle interior (10) while performing bacteria inactivation on filter (7).

Air conditioning system (2) according to a twelfth aspect further includes controller (8) in any one of the first to fifth aspects. Controller (8) performs drive control of air conditioning system (2). Controller (8) is configured to execute an exposure mode of operating blower (4) to output the air from blower (4) at a first air pressure and exposing evaporator (5) to the functional substance. After executing the exposure mode, controller (8) executes at least one of a drying mode of operating blower (4) to output air from blower (4) at a second air pressure higher than the first air pressure and drying evaporator (5) or a filling mode of filling case (20) with the functional substance.

In this aspect, bacteria inactivation on evaporator (5) can be performed more satisfactorily by further drying evaporator (5) or by further performing bacteria inactivation on evaporator (5) after bacteria inactivation on evaporator (5) is performed in the exposure mode.

Air conditioning system (2) according to a thirteenth aspect further includes, in any one of the first to fifth aspects, air duct (D3), vehicle-interior duct (D2), air duct valve (28), vehicle-interior valve (27), and controller (8). Air duct (D3) is disposed downstream of evaporator (5) in the first direction and connects the inside of vehicle interior (10) and case (20). Vehicle-interior duct (D2) connects air duct (D3) and functional substance generator (3). Air duct valve (28) opens and closes a flow path of air duct (D3). Vehicle-interior valve (27) opens and closes a flow path of vehicle-interior duct (D2). Controller (8) performs drive control of air conditioning system (2). Controller (8) is configured to execute an exposure mode of operating blower (4) to output the air from blower (4) at a first air pressure and exposing evaporator (5) to the functional substance. Controller (8) is configured to execute a drying mode of operating blower (4) to output the air from blower (4) at a second sir pressure higher than the first air pressure and drying evaporator (5). Controller (8) is configured to execute a filling mode of closing vehicle-interior valve (27) air duct valve (28) and filling case (20) with the functional substance. Controller (8) executes the exposure mode, the drying mode, and the filling mode in this order.

In this aspect, by drying evaporator (5) and further performing bacteria inactivation on evaporator (5) after performing bacteria inactivation on evaporator (5) in the exposure mode, bacteria inactivation on evaporator (5) can be more satisfactorily performed.

Configurations other than the first aspect are not essential for air conditioning system (2), and they can be omitted as appropriate.

### REFERENCE MARKS IN THE DRAWINGS

- 1: vehicle
- 10: vehicle interior
- 11: windshield
- 12: instrument panel
- 13: seat
- 14: foot space
- 15: device room
- 16: engine room
- 2: air conditioning system
- 20: case
- 201: junction port
- 202: junction port
- 21: defrost air outlet
- 22: face air outlet
- 23: foot air outlet
- 24: internal air introduction port
- 25: external air introduction port
- 26: case valve
- 261: valve
- 262: valve
- 27: vehicle-interior valve
- 28: air duct valve
- 291: introduction port valve
- 292: valve
- 293: valve
- 294: valve
- 295: heater valve
- 296: valve
- 297: valve
- 3: functional substance generator
- 4: blower
- 4a: second direction blower
- 5: evaporator
- 6: heater
- 7: filter
- 7a: filter
- 8: controller
- 9: operation unit
- D0: functional substance duct
- D1: case duct
- D2: vehicle-interior duct
- D3: air duct
- D4: duct
- D5: duct
- D6: bypass duct
- D7: internal-air duct
- D8: external-air duct
- D9: duct
- D11: first case duct
- D12: second case duct
- Sp1: space

## Claims

1. An air conditioning system comprising:
a blower configured to generate a flow of air toward a vehicle interior;
an evaporator configured to exchange heat of the air, the evaporator being disposed in a middle of a flow path of the air;
a case accommodating the blower and the evaporator; and
a functional substance generator configured to generate a functional substance,
wherein
the functional substance generator is disposed upstream of the evaporator in a first direction which is a flow direction of the air toward the vehicle interior.

2. The air conditioning system according to Claim 1, further comprising:
a case duct connecting the case to the functional substance generator,
wherein
the case includes a junction port located upstream of the evaporator in the first direction,
the functional substance generator is disposed outside the case, and
the functional substance is configured to be sent into the case through the case duct and the junction port.

3. The air conditioning system according to Claim 2, further comprising:
an air duct disposed downstream of the evaporator in the first direction, the air duct connecting the vehicle interior to the case; and
a vehicle-interior duct connecting the air duct to the functional substance generator,
wherein
the functional substance is configured to be sent into at least one of the vehicle interior or the case through the vehicle-interior duct.

4. The air conditioning system according to Claim 1, wherein
the functional substance generator is disposed in the case.

5. The air conditioning system according to Claim 4, wherein
the functional substance generator is disposed between the blower and the evaporator.

6. The air conditioning system according to any one of Claims 1 to 5, further comprising:
a controller configured to perform drive control of the air conditioning system,
wherein
the controller is configured to execute:
an exposure mode of operating the blower to output the air from the blower at a first air pressure and exposing the evaporator to the functional substance; and
a drying mode of operating the blower to output the air from the blower at a second air pressure higher than the first air pressure and drying the evaporator.

7. The air conditioning system according to any one of Claims 1 to 5, further comprising:
an air duct disposed downstream of the evaporator in the first direction, the air duct connecting the vehicle interior to the case;
an air duct valve configured to open and close a flow path of the air duct; and
a controller configured to perform drive control of the air conditioning system,
wherein
the controller is configured to execute a filling mode of closing the air duct valve and filling the case with the functional substance.

8. The air conditioning system according to Claim 7, wherein
the controller is configured to operate the blower to generate a flow of the air in a second direction which is opposite to the first direction after a lapse of a predetermined time from start of execution of the filling mode.

9. The air conditioning system according to any one of Claims 1 to 5, further comprising:
a heater disposed downstream of the evaporator in the first direction; and
a controller configured to perform drive control of the air conditioning system,
wherein
the controller is configured to execute a drying mode of operating the heater, operating the blower to generate a flow of the air in a second direction which is opposite to the first direction, and drying the evaporator.

10. The air conditioning system according to any one of Claims 1 to 5, further comprising:
a controller configured to perform drive control of the air conditioning system; and
a heater disposed downstream of the evaporator in the first direction; and
a second direction blower configured to generate a flow of the air in a second direction opposite to the first direction,
wherein
the controller is configured to execute a drying mode of operating the heater, operating the second direction blower without operating the blower, and drying the evaporator.

11. The air conditioning system according to Claim 8, further comprising:
an air duct disposed downstream of the evaporator in the first direction, the air duct connecting the vehicle interior to the case;
a vehicle-interior duct connecting the air duct to the functional substance generator; and
a vehicle-interior valve configured to open and close a flow path of the vehicle-interior duct,
wherein
the controller opens the vehicle-interior valve and operates the blower to generate a flow of the air in a second direction which is opposite to the first direction.

12. The air conditioning system according to any one of Claims 1 to 5, further comprising:
a controller configured to perform drive control of the air conditioning system,
wherein
the controller is configured to execute an exposure mode of operating the blower to output the air from the blower at a first air pressure and exposing the evaporator to the functional substance, and
the controller executes, after executing the exposure mode, at least one of a drying mode of operating the blower to output the air from the blower at a second air pressure higher than the first air pressure and drying the evaporator or a filling mode of filling the case with the functional substance.

13. The air conditioning system according to any one of Claims 1 to 5, further comprising:
an air duct disposed downstream of the evaporator in the first direction, the air duct connecting the vehicle interior to the case;
a vehicle-interior duct connecting the air duct to the functional substance generator;
an air duct valve configured to open and close a flow path of the air duct;
a vehicle-interior valve configured to open and close a flow path of the vehicle-interior duct; and
a controller configured to perform drive control of the air conditioning system,
wherein
the controller is configured to execute:
an exposure mode of operating the blower to output the air from the blower at a first air pressure and exposing the evaporator to the functional substance;
a drying mode of operating the blower to output the air from the blower at a second air pressure higher than the first air pressure and drying the evaporator; and
a filling mode of closing the vehicle-interior valve and the air duct valve to fill the case with the functional substance, and
the controller is configured to execute the exposure mode, the drying mode, and then the filling mode.
